# EUROPEAN PATENT SPECIFICATION

(11) **EP 1 503 701 B1**
(45) Date of publication and mention of the grant of the patent: **21.08.2013**
(21) Application number: 03741776.3
(22) Date of filing: 05.05.2003
(51) Int. Cl.: A61F 2/06

(54) **SHAPE MEMORY POLYMER STENT**
STENT AUS POLYMER MIT FORMGEDÄCHTNIS
STENT EN MATERIAU POLYMERE A MEMOIRE DE FORME

(30) Priority: 14.05.2002 US 145387
(43) Date of publication of application: 09.02.2005
(60) Divisional application: 12195744.3
(73) Proprietor: Micrus Endovascular Corporation, San Jose, CA 95131 (US)
(72) Inventor: DEBEER, Nicholas, C., Montana, CA 94037 (US); KURZ, Daniel, R., Monterey, CA 93940 (US); FERRERA, David, A., Manhattan Beach, CA 90266 (US)
(74) Representative: McLeish, Nicholas Alistair Maxwell
(86) International application number: PCT/US2003/014056
(87) International publication number: WO 2003/096934

(56) References cited:
- WO-A-99/42147
- US-A- 5 766 238
- US-A- 5 911 753
- US-A- 6 022 371
- US-A- 6 048 360
- US-A1- 2001 044 651

## Description

### BACKGROUND OF THE INVENTION

### Field of the Invention:

This invention relates generally to implantable devices for interventional therapeutic treatment or vascular surgery, and more particularly concerns a shape memory polymer stent.

### Description of Related Art:

The art and science of interventional therapy and surgery has continually progressed towards treatment of internal defects and diseases by use of ever smaller incisions or access through the vasculature or body openings in order to reduce the trauma to tissue surrounding the treatment site. One important aspect of such treatments involves the use of catheters to place therapeutic devices at a treatment site by access through the vasculature. Examples of such procedures include transluminal angioplasty, placement of stents to reinforce the walls of a blood vessel or the like and the use of vasoocclusive devices to treat defects in the vasculature. There is a constant drive by those practicing in the art to develop new and more capable systems for such applications. When coupled with developments in biological treatment capabilities, there is an expanding need for technologies that enhance the performance of interventional therapeutic devices and systems.

One specific field of interventional therapy that has been able to advantageously use recent developments in technology is the treatment of neurovascular defects. More specifically, as smaller and more capable structures and materials have been developed, treatment of vascular defects in the human brain which were previously untreatable or represented unacceptable risks via conventional surgery have become amenable to treatment.

Stents are typically implanted within a vessel in a contracted state and expanded when in place in the vessel in order to maintain patency of the vessel, and such stents are typically implanted by mounting the stent on a balloon portion of a balloon catheter, positioning the stent in a body lumen, and expanding the stent to an expanded state by inflating the balloon. The balloon is then deflated and removed, leaving the stent in place. However, the placement, inflation and deflation of a balloon catheter is a complicated procedure that involves additional risks beyond the implantation of the stent, so that it would be desirable to provide a stent that can be more simply placed in the site to be treated in a compressed state, and expanded to leave the stent in place.

A number of stents formed from polymeric memory materials are known that transform from a compressed configuration to an expanded configuration. One such conventional stent is known, for example, that provides a casing formed from a memory elastomer such as polyurethane, and a support structure that can be manufactured by braiding individual threads formed of a temperature-sensitive polyurethane that is hard below 25°C and that softens above 35°C, so that at a temperature slightly below body temperature, the stent changes from a pressed configuration to an expanded configuration.

However, stents formed of shape memory polymeric materials typically do not provide adequate structural and mechanical radial strength requirements for a stent. Stents are therefore commonly provided with a metallic structure to provide the strengtli required to function as a stent. It would therefore be desirable to provide a shape memory polymer stent having a configuration that would provide adequate structural and mechanical radial strength for a stent, and that can be deployed without requiring inflation and deflation of a balloon catheter, by pushing the stent in a compressed state for deployment at the site to be treated, where the stent can be expanded to leave the stent in place. It would also be desirable to provide a stent formed of a shape memory polymer that has a glass transition temperature (Tg) above body temperature to allow for a controlled transition from a compressed configuration to an expanded configuration when exposed to body temperature, by controlled heating of the stent. The present invention meets these and other needs.

US 6,048,360 discloses a prosthesis comprising an expandable coiled sheet with a biocompatible graft affixed thereto.

US 6,022,371 discloses a stent which may be mechanically locked in an expanded diameter. In use the locking stent may be mounted on a stent delivery catheter which incorporates a pull back sheath. Once the catheter is in position the sheath may be pulled back, thereby allowing the stent to expand. The catheter may then be removed and if desired a balloon catheter may be used to expand any unlocked cells of the locking stent.

### SUMMARY OF THE INVENTION

According to the invention there is provided a stent as defined in the claims.

The invention accordingly provides, in a first embodiment, for a shape memory polymer stent, comprising an extruded tube having a truss-like design, and formed from a polymer having shape memory properties. In one presently preferred aspect, the polymer can be a polyurethane that can be compressed from an originally expanded configuration with a predetermined shape to have a reduced diameter to fit into a catheter or delivery system, and that can return to its predetermined shape and original expanded diameter after heating of the stent above its glass transition temperature. The stent can, for example, be formed as an extruded tube, and processed to remove segments yielding a truss-like design for improved radial strength.

In a second embodiment, the invention provides for a shape memory polymer stent, comprising a tube woven from extruded strands of a polymer having shape memory properties. In one presently preferred aspect, the polymer can be a polyurethane that can be compressed from an originally expanded configuration with a predetermined shape to have a reduced diameter to fit into a catheter or delivery system, and that can return to its predetermined shape and original expanded diameter after heating of the stent above its glass transition temperature.

In each of the foregoing embodiments, after formation of the stent in its expanded configuration with a predetermined shape, by heating the stent above its glass transition temperature (Tg), the stent of shape memory material transitions into the rubbery state and can be compressed to be axially stressed in the distal direction to have a reduced diameter and increased length. In one presently preferred embodiment, the stent of the invention can be compressed over a mounting portion of a pusher catheter for deployment within the vasculature. In a preferred aspect, the outer diameter of the pusher member on either side of the stent is smaller than the inner diameter of the stent in its expanded configuration but greater than the inner diameter of the stent in its compressed configuration, while the outer diameter of the mounting portion of the pusher member over which the stent is placed has a reduced diameter that is less than or equal to the inner diameter of the stent in its compressed configuration.

In the elongated state, the stent can fit within a catheter or other delivery system for delivery through the vasculature. Because the stent is formed from a shape memory material, it will return to its original shape and dimensions to relieve the external stress of compression, if allowed to remain above T_{g}. However, before the stent can recover its original shape and dimensions, it can be fixed in the compressed, elongated configuration and mounted over the mounting portion of the pusher catheter by lowering the temperature of the material below T_{g}. The stent can then be inserted into the vasculature and maneuvered into a desired location mounted on the pusher catheter, and heat can be transferred to the stent from the pusher catheter, such as by transmission of light energy, through a heat pipe, by conducting electricity through electrical resistance, transmission of radio-frequency electro-magnetic waves or ultra-sonic waves, or other means. The heat transfer causes the temperature of the stent to once again rise above T_{g} and causes the stent to transition back into the rubbery state to radially expand and axially retract to its original shape and dimensions, deploying the stent in the vasculature and allowing the pusher catheter to be retracted from the vasculature.

These and other aspects and advantages of the invention will become apparent from the following detailed description and the accompanying drawings, which illustrate by way of example the features of the invention.

### BRIEF DESCRIPTION OF THE DRAWINGS

FIGURE 1 is a plan view of a first embodiment of the stent of the invention formed from an extruded tube and processed to remove segments yielding a truss-like design, in an original expanded configuration in a predetermined shape.
Fig. 2 is an end view of the stent of Fig. 1.
Fig. 3 is a perspective view of the stent of Fig. 1.
Fig. 4 is a perspective view of the stent of Fig. 1 in a compressed, elongated configuration.
Fig. 5 is a plan view of a second embodiment of the stent of the invention woven from extruded strands, in an original expanded configuration in a predetermined shape.
Fig. 6 is an end view of the stent of Fig. 5.
Fig. 7 is a perspective view of the stent of Fig. 5.
Fig. 8 is a perspective view of the stent of Fig. 5 in a compressed, elongated configuration.
Fig. 9 is a plan view of the stent of Fig. 1 in a compressed, elongated configuration and mounted over a pusher catheter for placement in the vasculature.
Fig. 10 is a plan view of the stent of Fig. 1 in a compressed, elongated configuration and mounted over a pusher catheter for placement in the vasculature.
Fig. 11 is a plan view of the stent of Fig. 1 showing the stent in its expanded configuration deployed in the vasculature, and allowing retraction of a pusher catheter.
Fig. 12 is a plan view of the stent of Fig. 5 in a compressed, elongated configuration and mounted over the pusher catheter of Fig. 9 for placement in the vasculature.
Fig. 13 is a plan view of the stent of Fig. 5 showing the stent in its expanded configuration deployed in the vasculature, and allowing retraction of a pusher catheter.

### DETAILED DESCRIPTION OF THE PREFERRED EMBODIMENTS

While stents formed from polymeric memory materials are known that transform from a compressed configuration to an expanded configuration, stents formed of shape memory polymeric materials typically do not provide adequate structural and mechanical radial strength requirements for a stent, and in the past have been formed of a shape memory polymer having a glass transition temperature (Tg) below body temperature, making the transition from a compressed configuration to an expanded configuration more difficult to control when the stent is exposed to body temperature.

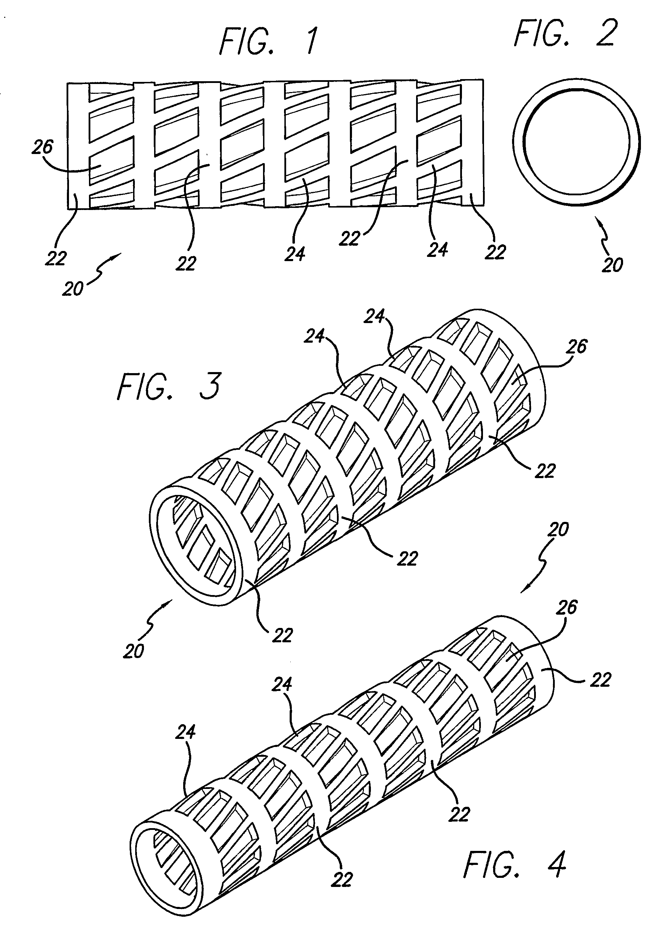

As is illustrated in the drawings, the invention is embodied in a shape memory polymer stent for treatment of a target site in a body lumen, or an intravascular flow modifier (IFM) having a tubular framework, for use in treating aneurysms such as cerebral or abdominal aneurysms. Referring to Figs. 1-4, in one presently preferred embodiment, the shape memory polymer stent or IFM 20, having a tubular framework, is preferably formed from a plurality of annular support members 22 and a plurality of cross-struts 24 intersecting the plurality of annular support members. Referring to Figs. 1 and 2, the shape memory polymer stent can be formed from an extruded tube, and can be processed to remove segments, such as by cutting a plurality of openings 26 in the extruded tube with a laser, for example, to form the intersecting annular support members and the plurality of cross-struts, providing a truss-like design. In a presently preferred embodiment, illustrated in Fig. 3, the shape memory polymer is a polyurethane that can take a predetermined shape having an expanded diameter, such as 3 mm. for example, after heating above its Tg (glass transition temperature), and a reduced diameter, shown in Fig. 4, such as of about 1 mm., for example, to fit into a catheter or delivery system. In a presently preferred embodiment illustrated in Figs. 1, 3 and 4, the cross-struts are formed to extend at an oblique angle relative to the plurality of annular support members. Alternatively, the cross-struts could be formed at other angles, such as to intersect orthogonally with the annular support members, for example.

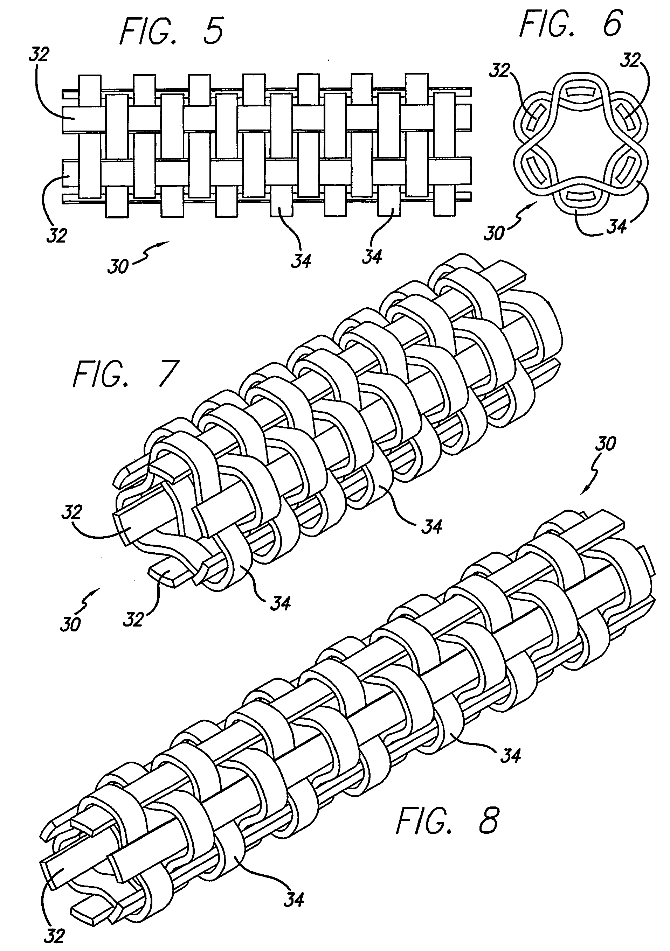

Referring to Figs. 5-8, in a second preferred embodiment, the present invention provides for a woven stent or intravascular flow modifier (IFM) 30 that can be woven from extruded strands to form a shape memory polymer stent or IFM having a tubular framework. Referring to Figs. 5 and 6, the woven shape memory polymer stent can be woven from extruded strands forming a longitudinal warp of cross-struts 32 and an annular woof of support members 34 forming orthogonally intersecting strands. Alternatively, the woven shape memory polymer stent or IFM can be woven from extruded strands forming a longitudinal warp and a spiral woof of intersecting strands. In a presently preferred embodiment, the-woven shape memory polymer stent is formed from polyurethane that can take a predetermined shape, shown in Fig. 7, having an expanded diameter, such as 3 mm. for example, after heating above its Tg (glass transition temperature), and a reduced diameter, shown in Fig. 8, such as of about 1 mm. for example, to fit into a catheter or delivery system.

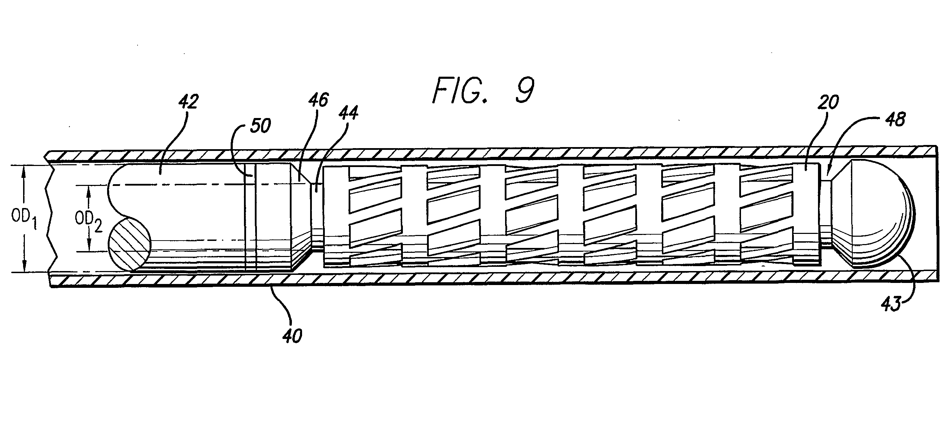

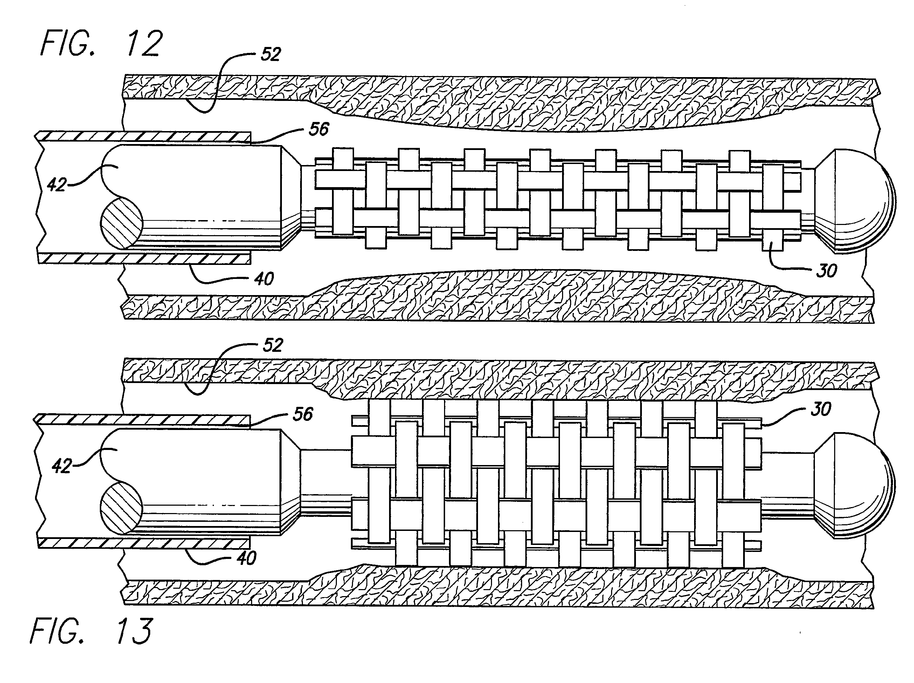

Referring to Figs. 9 and 12, the extruded shape memory polymer stent or IFM 20, or the woven shape memory polymer stent or IFM 30, can be introduced through an introducer catheter into a target site of a blood vessel to be treated in a compressed, elongated configuration, by mounting the stent over an elongated pusher catheter or pusher member 42 having a distal end 43, for placement in the vasculature. The proximal end of the pusher member is not shown, for simplicity. The pusher member can be formed from a fiber optic member, having an inner optical conductor portion 44, and an outer buffer layer 46. As is illustrated in Fig. 9, the pusher member preferably has a principal outer diameter (OD1) over the majority of the length of the elongated pusher member, and a distal region of the fiber optic member having at least a portion of outer buffer layer removed to provide a distal seating region 48 having a recessed outer diameter (OD2) that is less than the principal outer diameter, over which the shape memory polymer stent can be mounted. In a presently preferred embodiment, as is illustrated in Fig. 9, one or more radiopaque markers 50 may also be provided on the pusher member.

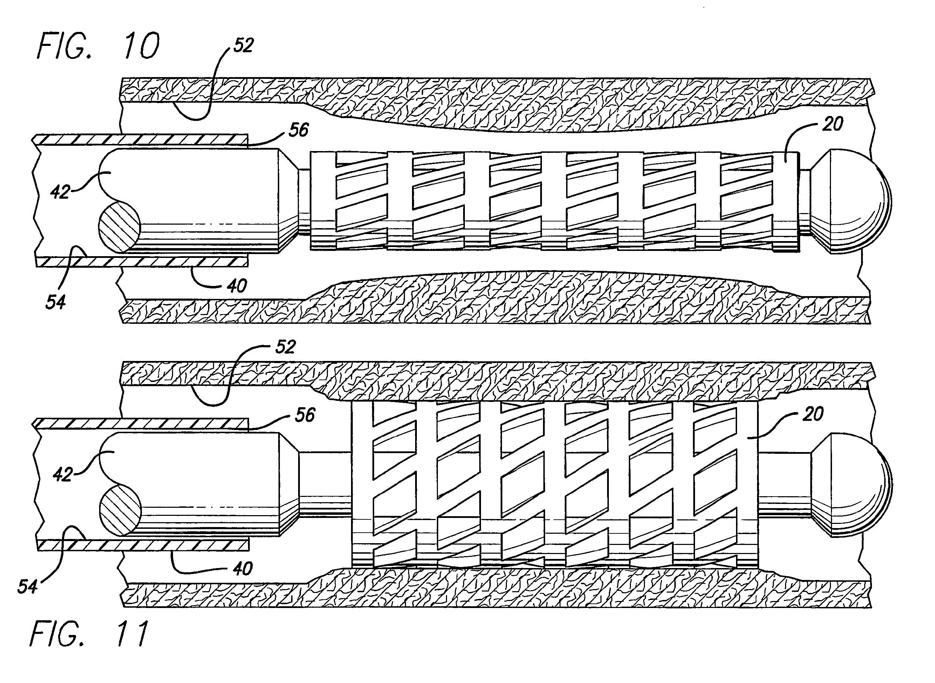

Referring to Figs. 10 and 12, in a compressed, elongated configuration mounted over a pusher member for placement in the vasculature, an extruded tubular shape memory polymer stent or IFM 20, or a woven shape memory polymer stent or IFM 30, can be placed in a body lumen such as a blood vessel 52 at a target location of a stenosis by introducing the distal seating portion of the elongated pusher member and tubular shape memory polymer stent mounted thereon into a lumen 54 of the introducer catheter, positioning the catheter within the blood vessel or other body lumen so that the distal opening of the catheter is proximal to the target site to be treated, and pushing the distal seating portion of the elongated pusher member carrying the tubular shape memory polymer stent out of the distal opening 56 of the catheter to the target site to be treated. As is illustrated in Figs. 11 and 13, the extruded or woven tubular shape memory polymer stent or IFM can be heated to cause the shape memory polymer stent or IFM to transition to the expanded configuration, thereby deploying the tubular shape memory polymer stent within the target site of the blood vessel or body lumen, or within an aneurysm and at least partially occluding the opening between the aneurysm and the parent blood vessel, and allowing retraction of a pusher member. The shape memory polymer stent or IFM can be heated by causing energy to be transmitted through the elongated pusher member to release the connection between the pusher member and the shape memory polymer stent or IFM. In a presently preferred embodiment, the pusher member comprises a fiber optic member, so that the tubular shape memory polymer stent can be heated by conducting light energy through the fiber optic member to the seating region of the elongated pusher member to heat the shape memory polymer stent or IFM. Alternatively, the elongated pusher member can be a heat pipe, and the shape memory polymer stent or IFM can be heated by conducting heat along the heat pipe elongated pusher member to the seating region of the elongated pusher member to heat the tubular shape memory polymer stent. In another alternate embodiment, the shape memory polymer stent or IFM can be heated by heating the shape memory polymer stent or IFM by conducting electricity through electrical resistance, transmission of radio-frequency electro-magnetic waves (RF) or ultra-sonic waves, or other similar means.

It will be apparent from the foregoing that while particular forms of the invention have been illustrated and described, various modifications can be made without departing from the scope of the invention. Accordingly, it is not intended that the invention be limited, except as by the appended claims.

## Claims

1. A shape memory polymer stent having a tubular framework (20), comprising:
a plurality of annular support members (22, 34); and
a plurality of cross struts (24, 32) intersecting said plurality of annular support members, the shape memory polymer stent having a compressed configuration at a temperature below body temperature and an enlarged configuration at a temperature above body temperature and being **characterised in that**;
the shape memory polymer stent has a glass transition temperature that is above body temperature.

2. The shape memory polymer stent of Claim 1, wherein said tubular framework (20) is formed from an extruded tube having a surface defining said plurality of annular support members (22), said plurality of cross-struts (24), and a plurality of openings (26) between said plurality of annular support members (22) and said plurality of cross-struts (24).

3. The shape memory polymer stent of Claim 1, wherein said plurality of cross-struts (24) extend at an oblique angle relative to said plurality of annular support members (22).

4. The shape memory polymer stent of Claim 2, wherein said extruded tube is formed of a polymer having shape memory properties.

5. The shape memory polymer stent of Claim 4, wherein said polymer is polyurethane.

6. The shape memory polymer stent of Claim 1, wherein said tubular framework (30) is formed from woven strands of said plurality of annular support members (34) and said plurality of cross-struts (32).

7. The shape memory polymer stent of claim 6, wherein said plurality of annular support members form an annular woof, and said plurality of cross-struts (32) extend longitudinally and form a longitudinal warp.

## Patentansprüche

1. Formgedächtnispolymer-Stent mit einer röhrenförmigen Rahmenstruktur (20), der aufweist:
mehrere ringförmige Stützelemente (22, 34); und
mehrere Querverstrebungen (24, 32), die die mehreren ringförmigen Stützelemente kreuzen, wobei der Formgedächtnispolymer-Stent bei einer Temperatur unterhalb der Körpertemperatur eine komprimierte Gestalt und bei einer Temperatur über der Körpertemperatur eine aufgeweitete Gestalt aufweist und **dadurch gekennzeichnet ist, dass**
der Formgedächtnispolymer-Stent eine oberhalb der Körpertemperatur gelegene Glasübergangstemperatur aufweist.

2. Formgedächtnispolymer-Stent nach Anspruch 1, worin die röhrenförmige Rahmenstruktur (20) aus einer extrudierten Röhre gebildet ist, deren Oberfläche die mehreren ringförmigen Stützelemente (22), die mehreren Querverstrebungen (24) und zwischen den mehreren ringförmigen Stützelementen (22) und den mehreren Querverstrebungen (24) mehrere Öffnungen (26) definiert.

3. Formgedächtnispolymer-Stent nach Anspruch 1, worin sich die mehreren Querverstrebungen (24) relativ zu den mehreren ringförmigen Stützelementen (22) in einem schrägen Winkel erstrecken.

4. Formgedächtnispolymer-Stent nach Anspruch 2, worin die extrudierte Röhre aus einem Polymer mit Formgedächtniseigenschaften gebildet ist.

5. Formgedächtnispolymer-Stent nach Anspruch 4, worin das Polymer Polyurethan ist.

6. Formgedächtnispolymer-Stent nach Anspruch 1, worin die röhrenförmige Rahmenstruktur (30) aus gewebten Strängen der mehreren ringförmigen Stützelemente (34) und der mehreren Querverstrebungen (32) gebildet ist.

7. Formgedächtnispolymer-Stent nach Anspruch 6, worin die mehreren ringförmigen Stützelemente einen ringförmigen Schuss bilden und die mehreren Querverstrebungen (32) sich in Längsrichtung erstrecken und eine längsverlaufende Kette bilden.

## Revendications

1. Extenseur en polymère à mémoire de forme, ayant un châssis tubulaire (20), comprenant :
une pluralité d'éléments de support annulaires (22, 34) ; et
une pluralité de croisillons (24, 32) se croisant avec ladite pluralité d'éléments de support annulaires, l'extenseur en polymère à mémoire de forme ayant une configuration compressée à une température en dessous de la température corporelle et une configuration agrandie à une température au-dessus de la température corporelle et étant **caractérisé en ce que** :
l'extenseur en polymère à mémoire de forme présente une température de transition vitreuse qui est au-dessus de la température corporelle.

2. Extenseur en polymère à mémoire de forme selon la revendication 1, où ledit châssis tubulaire (20) est formé à partir d'un tube extrudé ayant une surface définissant ladite pluralité d'éléments de support annulaires (22), ladite pluralité de croisillons (24) et une pluralité d'ouvertures (26) entre ladite pluralité d'éléments de support annulaires (22) et ladite pluralité de croisillons (24).

3. Extenseur en polymère à mémoire de forme selon la revendication 1, où ladite pluralité de croisillons (24) s'étend suivant un angle oblique relativement à ladite pluralité d'éléments de support annulaires (22).

4. Extenseur en polymère à mémoire de forme selon la revendication 2, où ledit tube extrudé est réalisé en un polymère ayant des propriétés de mémoire de forme.

5. Extenseur en polymère à mémoire de forme selon la revendication 4, où ledit polymère est le polyuréthane.

6. Extenseur en polymère à mémoire de forme selon la revendication 1, où ledit châssis tubulaire (30) est formé à partir de torons tissés de ladite pluralité d'éléments de support annulaires (34) et de ladite pluralité de croisillons (32).

7. Extenseur en polymère à mémoire de forme selon la revendication 6, où ladite pluralité d'éléments de support annulaires forme une trame annulaire, et ladite pluralité de croisillons (32) s'étendent longitudinalement et forment une chaîne longitudinale.
